# FASCICULE DE BREVET EUROPEEN

(11) **EP 4 157 521 B1**
(45) Date de publication et mention de la délivrance du brevet: **04.03.2026**
(21) Numéro de dépôt: 21726389.6
(22) Date de dépôt: 18.05.2021
(51) Int. Cl.: B01J 23/755, B01J 21/04, B01J 35/30, B01J 37/02, C10G 45/36, C10G 45/48, C07C 5/05, C07C 5/10, C07C 5/03

(54) **PROCEDE DE PREPARATION D'UN CATALYSEUR COMPRENANT UNE PHASE ACTIVE DE NICKEL REPARTIE EN CROUTE**
VERFAHREN ZUR HERSTELLUNG EINES KATALYSATORS MIT EINER IN EINER SCHALE VERTEILTEN AKTIVEN NICKELPHASE
METHOD FOR PREPARING A CATALYST COMPRISING AN ACTIVE NICKEL PHASE DISTRIBUTED IN A SHELL

(30) Priorité: 29.05.2020 FR 2005680
(43) Date de publication de la demande: 05.04.2023
(73) Titulaire: IFP Energies nouvelles, 92500 Rueil-Malmaison (FR)
(72) Inventeur: BOUALLEG, Malika, 92852 RUEIL-MALMAISON CEDEX (FR)
(74) Mandataire: IFP Energies nouvelles
(86) Numéro de dépôt international: PCT/EP2021/063046
(87) Numéro de publication internationale: WO 2021/239497

(56) Documents cités:
- WO-A1-2021/018604
- US-A1- 2012 065 442
- US-A1- 2015 099 622
- JANG MIN-SU ET AL: "Facile preparation of egg-shell-type pellet catalysts using immiscibility between hydrophobic solvent and hydrophilic solution: Enhancement of catalytic activity due to position control of metallic nickel inside alumina pellet", APPLIED CATALYSIS A: GENERAL, ELSEVIER, AMSTERDAM, NL, vol. 530, 16 November 2016 (2016-11-16), pages 211 - 216, XP029858638, ISSN: 0926-860X, DOI: 10.1016/J.APCATA.2016.11.025
- FUJITANI T ET AL: "The Effect of Starting Source on the Concentration Distribution of Nickel Supported on Alumina", BULLETIN OF THE CHEMICAL SOCIETY OF JAPAN, CHEMICAL SOCIETY OF JAPAN, TOKYO, JP, vol. 62, 1 January 1989 (1989-01-01), pages 2753 - 2755, XP009120008, ISSN: 0009-2673, DOI: 10.1246/BCSJ.62.2753

## Description

### Domaine technique

La présente invention concerne un procédé de préparation d'un catalyseur métallique supporté à base de nickel destiné particulièrement à l'hydrogénation des hydrocarbures insaturés, et plus particulièrement, d'hydrogénation sélective de composés polyinsaturés ou d'hydrogénation des aromatiques.

### Etat de la technique

Les composés organiques mono-insaturés tels que par exemple l'éthylène et le propylène, sont à la source de la fabrication de polymères, de matières plastiques et d'autres produits chimiques à valeur ajoutée. Ces composés sont obtenus à partir du gaz naturel, du naphta ou du gazole qui ont été traités par des procédés de vapocraquage ou de craquage catalytique. Ces procédés sont opérés à haute température et produisent, en plus des composés mono-insaturés recherchés, des composés organiques polyinsaturés tels que l'acétylène, le propadiène et le méthylacétylène (ou propyne), le 1-2-butadiène et le 1-3-butadiène, le vinylacétylène et l'éthylacétylène, et d'autres composés polyinsaturés dont le point d'ébullition correspond à la fraction essence C5+ (essences contenant des composés hydrocarbonés ayant 5 atomes de carbone ou plus), en particulier des composés styréniques ou indéniques. Ces composés polyinsaturés sont très réactifs et conduisent à des réactions parasites dans les unités de polymérisation. Il est donc nécessaire de les éliminer avant de valoriser ces coupes. L'hydrogénation sélective est le principal traitement développé pour éliminer spécifiquement les composés polyinsaturés indésirables de ces charges d'hydrocarbures. Elle permet la conversion des composés polyinsaturés vers les alcènes ou aromatiques correspondants en évitant leur saturation totale et donc la formation des alcanes ou naphtènes correspondants.

Les catalyseurs d'hydrogénation sélective sont généralement à base de métaux du groupe VIII du tableau périodique, de préférence le palladium ou le nickel. Le métal se présente sous la forme de particules métalliques déposées sur un support. La teneur en métal, la taille des particules de métal et la répartition de la phase active dans le support font partie des critères qui ont une importance sur l'activité et la sélectivité des catalyseurs.

La répartition macroscopique des particules métalliques dans le support constitue un critère important, principalement dans le cadre de réactions rapides et consécutives telles que les hydrogénations sélectives. Il est généralement souhaitable que ces éléments se situent dans une croûte à la périphérie du support afin d'éviter les problèmes de transfert de matière intragranulaire pouvant conduire à des défauts d'activité et une perte de sélectivité. De tels catalyseurs sont aussi appelé catalyseurs "eggshell" selon la terminologie anglo-saxonne. De tels catalyseurs sont largement connus dans le cas des catalyseurs d'hydrogénation sélective à base de palladium. En effet, grâce à la faible teneur en palladium (généralement inférieure à 1 % en poids (1 % pds) de palladium par rapport au catalyseur) et des procédés de préparation adaptés, une croûte fine de palladium à la périphérie des grains de support peut être obtenue (FR2922784, US2010/217052).

Il est souvent proposé de substituer le palladium par le nickel, métal moins actif que le palladium qu'il est donc nécessaire de disposer en plus grande quantité dans le catalyseur. Ainsi, les catalyseurs à base de nickel ont généralement une teneur en métal entre 5 et 50 % pds de nickel par rapport au catalyseur. Dans ces catalyseurs, le nickel est généralement réparti de façon homogène au sein du support. Une des voies d'amélioration possible de ces catalyseurs en termes d'activité et de sélectivité est de contrôler la répartition du nickel au sein du support en déposant le nickel de façon plus concentrée sur une croûte, à la périphérie du support. De tels catalyseurs sont connus de l'état de l'art.

Le document US 4 519 951 décrit un catalyseur de type « eggshell » avec du nickel sur un support poreux ayant un volume poreux des pores dont la taille est inférieure à 11,7 nm d'au moins 0,2 ml/g et un volume poreux des pores dont la taille est supérieure à 11,7 nm d'au moins 0,1 ml/g. Plus de 50 % poids du nickel se trouve dans une croûte dont l'épaisseur est égale à 0,15 fois le rayon du support. Ce catalyseur est utilisé pour l'hydrogénation de matières grasses.

Le document CN101890351 décrit un catalyseur supporté de nickel dans lequel plus de 90 % poids du nickel se trouve dans une croûte de 700 µm d'épaisseur. Le catalyseur est préparé en utilisant une solution ammoniacale pour dissoudre le sel de nickel. Ces catalyseurs sont utilisés dans une application d'hydrogénation sélective.

Le document US2012/0065442 décrit un catalyseur supporté de nickel reparti à la fois sur une croûte d'une épaisseur de 3 à 15 % du diamètre et à coeur, le ratio de concentration en nickel entre la croûte et le cœur étant compris entre 3,0 : 1 et 1,3 : 1. Le dépôt de la phase active de nickel est réalisé par pulvérisation (« *spray coating* » selon la terminologie anglo-saxonne) d'une solution ammoniacale d'un sel de nickel sur le support.

Le document de Jang Min-Su et al. intitulé : « Facile preparation of egg-shell-type pellet catalysts using immiscibility between hydrophobic solvent and hydrophilic solution: Enhancement of catalytic activity due to position control of metallic nickel inside alumina pellet », APPLIED CATALYSIS A: GENERAL, 530(2017), 211-216, divulgue un procédé de préparation d'un catalyseur contenant 5 % en poids de nickel sur un support d'alumine, avec une répartition du nickel concentrée dans un croûte mais aussi présent à cœur du support. Le procédé utilisé comprend une étape de mise en contact du support avec un alcool, tel que le butanol, éventuellement une étape de chauffage à une température au-dessus de la température d'ébullition de l'alcool, une étape d'imprégnation du support avec une solution de nickel, puis une étape de séchage à 150°C et enfin une étape de calcination.

La demande de brevet français déposée sous le n°19/08.719 par la Demanderesse décrit un procédé de préparation d'un catalyseur à base de nickel sur un support d'alumine obtenu selon une méthode bien spécifique, le nickel étant réparti à la fois sur une croûte en périphérie du support, et à cœur du support, l'épaisseur de ladite croûte étant comprise entre 2% et 15% du diamètre du catalyseur. Le procédé de préparation d'un tel catalyseur nécessite d'une part l'utilisation d'un support d'alumine spécifique ayant subi un traitement hydrothermal en présence d'une solution acide, et d'autre part la réalisation d'une étape de traitement hydrothermal après l'ajout d'un additif organique spécifique sur le précurseur de catalyseur.

### Objets de l'invention

La présente invention concerne ainsi un nouveau procédé de préparation d'un catalyseur qui permet l'obtention d'un catalyseur comprenant des performances au moins aussi bonnes, voire meilleures, en terme d'activité et de sélectivité dans le cadre des réactions d'hydrogénation sélective de composés polyinsaturés ou d'hydrogénation des aromatiques, tout en utilisant une quantité de phase de nickel effective inférieure (c'est-à-dire une quantité de nickel se trouvant in-fine en croute en périphérie du support permettant la réalisation des réactions d'hydrogénation sélective ou d'hydrogénation des aromatiques) à celle utilisée typiquement dans l'état de la technique, grâce notamment à une meilleure répartition de la phase active de nickel dans le support, rendant cette dernière plus accessible aux réactifs.

La présente invention a pour objet un procédé de préparation d'un catalyseur comprenant une phase active à base de nickel et un support d'alumine, ledit catalyseur comprenant entre 1 et 50% en poids de nickel élémentaire par rapport au poids total du catalyseur, le nickel étant réparti à la fois sur une croûte en périphérie du support, et à cœur du support, l'épaisseur de ladite croûte étant comprise entre 2% et 15% du diamètre du catalyseur, la taille des particules de nickel dans le catalyseur, mesurée sous forme oxyde, étant comprise entre 7 nm et 25 nm, lequel procédé comprend les étapes suivantes :
a) on imprègne ledit support avec un volume V1 d'une solution de butanol compris entre 0,2 et 0,8 fois le volume poreux total VPT dudit support pour obtenir un support imprégné ;
b) on laisse maturer le support imprégné obtenu à l'issue de l'étape a) pendant 0,5 heure à 40 heures ;
c) on imprègne le support imprégné maturé obtenu à l'issue de l'étape b) avec une solution comprenant au moins un précurseur de la phase active de nickel pour obtenir un précurseur de catalyseur ;
d) on sèche le précurseur de catalyseur obtenu à l'issue de l'étape c) à une température inférieure à 250°C.

De préférence, à l'étape c), le volume V2 de la solution comprenant au moins un précurseur de la phase active de nickel imprégné sur le support imprégné maturé obtenu à l'issue de l'étape b) est tel que le V2 = VPT - V1.

De manière surprenante, la Demanderesse a découvert que la réalisation d'une étape particulière d'imprégnation d'une solution de butanol sur un support poreux d'alumine, quel que soit son origine, suivie d'une étape de maturation avant l'étape d'ajout du précurseur de la phase active de nickel sur ledit support imprégné maturé, et cela sans réaliser d'étape de séchage intermédiaire entre l'imprégnation du butanol et l'imprégnation du précurseur de la phase active de nickel, permet d'obtenir un catalyseur dans lequel au moins une partie du nickel est répartie sur une croûte à la périphérie du support, l'autre partie du nickel étant répartie au cœur du catalyseur. Sans vouloir être lié par une quelconque théorie, la présence de butanol limite la migration de la phase active de nickel au cœur du support. En effet, une partie seulement de la porosité est occupée par le butanol. L'étape de maturation permet à la solution de butanol de migrer à cœur du support et de libérer une « couronne de pores libres » à la périphérie du support accessible par le nickel lors de l'étape d'imprégnation du précurseur de la phase active. De plus, le butanol et l'eau étant peu miscibles, la couche de butanol constitue une barrière limitant la diffusion du nickel à cœur du support.

De préférence, l'étape b) est réalisée à une température inférieure ou égale à 60°C.

De préférence, à l'étape a), on utilise une solution de n-butanol.

De préférence, l'étape d) est réalisée pendant un temps compris entre 0,5 heures et 12 heures.

De préférence, le procédé comprend en outre une étape e) dans laquelle on calcine le catalyseur obtenu à l'issue de l'étape d) à une température comprise entre 250°C et 600°C.

De préférence, l'étape e) est réalisée pendant 0,5 heure à 24 heures.

De préférence, à l'étape a), ledit volume V1 de ladite solution de butanol est compris entre 0,25 et 0,75 fois le volume poreux total VPT dudit support.

De préférence, le précurseur de la phase active de nickel est le nitrate de nickel, le chlorure de nickel, l'acétate de nickel ou le hydroxycarbonate de nickel.

De préférence, le catalyseur comprend une surface spécifique comprise entre 10 m²/g et 350 m²/g.

### Description de la figure

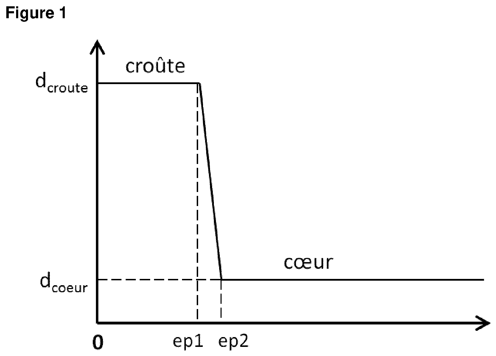

La figure 1 est un schéma représentant la répartition du nickel dans le catalyseur. L'axe des abscisses correspond à l'épaisseur du catalyseur, mesurée depuis le bord du catalyseur (en µm). L'axe des ordonnées correspond à la densité en nickel (en gramme de Ni / mm³). Le nickel est réparti à la fois sur une croûte en périphérie du support, d'épaisseur ep1, et à cœur du support. La densité en nickel sur la croûte d_{croute} est supérieure à la densité en nickel au cœur du support d_{coeur}. L'intervalle de transition entre le cœur et la croûte du catalyseur a une épaisseur notée ep2-ep1.

### Description détaillée de l'invention

### 1. Définitions

Dans la suite, les groupes d'éléments chimiques sont donnés selon la classification CAS (CRC Handbook of Chemistry and Physics, éditeur CRC press, rédacteur en chef D.R. Lide, 81ème édition, 2000-2001). Par exemple, le groupe VIII selon la classification CAS correspond aux métaux des colonnes 8, 9 et 10 selon la nouvelle classification IUPAC.

Dans la présente description, on entend, selon la convention IUPAC, par micropores les pores dont le diamètre est inférieur à 2 nm, c'est à dire 0,002 µm; par mésopores les pores dont le diamètre est supérieur ou égal à 2 nm, c'est à dire 0,002 µm et inférieur ou égal à 50 nm, c'est à dire 0,05 µm et par macropores les pores dont le diamètre est supérieur à 50 nm, c'est à dire 0,05 µm.

Afin d'analyser la répartition de la phase métallique sur le support, on mesure une épaisseur de croûte par microsonde de Castaing (ou microanalyse par microsonde électronique). L'appareil utilisé est un CAMECA XS100, équipé de quatre cristaux monochromateurs permettant l'analyse simultanée de quatre éléments. La technique d'analyse par microsonde de Castaing consiste en la détection de rayonnement X émis par un solide après excitation de ses éléments par un faisceau d'électrons de hautes énergies. Pour les besoins de cette caractérisation, les grains de catalyseur sont enrobés dans des plots de résine époxy. Ces plots sont polis jusqu'à atteindre la coupe au diamètre des billes ou extrudés puis métallisés par dépôt de carbone en évaporateur métallique. La sonde électronique est balayée le long du diamètre de cinq billes ou extrudés pour obtenir le profil de répartition moyen des éléments constitutifs des solides. Cette méthode, bien connue de l'Homme du métier, est définie dans la publication de L. Sorbier et al. "Measurement of palladium crust thickness on catalyst by EPMA" Materials Science and Engineering 32 (2012). Elle permet d'établir le profil de répartition d'un élément donné, ici le nickel, au sein du grain. Par ailleurs, la concentration en Ni est définie pour chaque mesure et donc pour chaque pas d'analyse. La densité de Ni au sein du grain est donc définie comme la concentration de Ni par mm³.

Le volume poreux total est mesuré par porosimétrie au mercure selon la norme ASTM D4284-92 avec un angle de mouillage de 140°, par exemple au moyen d'un appareil modèle Autopore III^{™} de la marque Microméritics^{™}.

La surface spécifique BET est mesurée par physisorption à l'azote selon la norme ASTM D3663-03, méthode décrite dans l'ouvrage Rouquerol F.; Rouquerol J.; Singh K. « Adsorption by Powders & Porous Solids: Principle, methodology and applications », Academic Press, 1999.

On définit également le diamètre médian mésoporeux comme étant le diamètre tel que tous les pores, parmi l'ensemble des pores constituant le volume mésoporeux, de taille inférieure à ce diamètre constituent 50% du volume mésoporeux total déterminé par intrusion au porosimètre à mercure.

On entend par « taille des particules de nickel » le diamètre des cristallites de nickel sous forme oxyde. Le diamètre des cristallites de nickel sous forme oxyde est déterminé par diffraction des rayons X, à partir de la largeur de la raie de diffraction située à l'angle 2thêta=43° (c'est-à-dire selon la direction cristallographique [200]) à l'aide de la relation de Scherrer. Cette méthode, utilisée en diffraction des rayons X sur des poudres ou échantillons polycristallins qui relie la largeur à mi-hauteur des pics de diffraction à la taille des particules, est décrite en détail dans la référence : Appl. Cryst. (1978), 11, 102-113 « Scherrer after sixty years: A survey and some new results in the determination of crystallite size», J. I. Langford and A. J. C. Wilson.

La teneur en nickel est mesurée par fluorescence X.

### 2. Procédé de préparation du catalyseur

La présente invention a pour objet un procédé de préparation d'un catalyseur comprenant une phase active à base de nickel et un support d'alumine, ledit catalyseur comprenant entre 1 et 50% en poids de nickel élémentaire par rapport au poids total du catalyseur, le nickel étant réparti à la fois sur une croûte en périphérie du support, et à cœur du support, l'épaisseur de ladite croûte étant comprise entre 2% et 15% du diamètre du catalyseur, la taille des particules de nickel dans le catalyseur, mesurée sous forme oxyde, étant comprise entre 7 nm et 25 nm, lequel procédé comprend les étapes suivantes :
a) on imprègne ledit support avec un volume V1 d'une solution de butanol compris entre 0,2 et 0,8 fois le volume poreux total VPT dudit support pour obtenir un support imprégné ;
b) on laisse maturer le support imprégné obtenu à l'issue de l'étape a) pendant 0,5 à 40 heures ;
c) on imprègne le support imprégné maturé obtenu à l'issue de l'étape b) avec une solution comprenant au moins un précurseur de la phase active de nickel pour obtenir un précurseur de catalyseur ;
d) on sèche le précurseur de catalyseur obtenu à l'issue de l'étape c) à une température inférieure à 250°C.

L'ordre des étapes de a) à d) n'est pas permutable. Cependant, il est possible d'ajouter des étapes supplémentaires avant utilisation du catalyseur à l'issue de l'étape d).

Les étapes dudit procédé de préparation sont décrites en détail ci-après.

### Etape a)

Selon l'étape a) du procédé, on imprègne le support d'alumine avec un volume V1 d'une solution de butanol compris entre 0,2 et 0,8 fois le volume poreux total (appelé aussi ici VPT) dudit support à imprégner, de préférence entre 0,25 et 0,75.

Par butanol, on entend les composés organiques comprenant une fonction alcool répondant à la formule chimique brute C₄H₁₀O. On entend ainsi par butanol la famille des composés organiques suivants : le butan-1-ol (ou n-butanol), le butan-2-ol, l'isobutanol, et le tert-butanol. De préférence l'étape a) est réalisée en présence de butan-1-ol.

### Etape b)

Après l'étape a), le support imprégné est maturé à l'état humide pendant 0,5 heure à 40 heures, de manière préférée pendant 1 heure à 30 heures. L'étape b) de maturation est de préférence réalisée à une température inférieure ou égale à 60°C, et plus préférentiellement à température ambiante. Cette étape permet la migration de la solution de butanol au cœur du support.

### Etape c)

Lors de l'étape c) du procédé, on imprègne le support poreux d'alumine imprégné maturé obtenu à l'issue de l'étape b) avec une solution comprenant au moins un précurseur de la phase active de nickel pour obtenir un précurseur de catalyseur. L'étape d'imprégnation est réalisée selon des méthodes bien connues de l'Homme du métier.

De préférence, le volume V2 de la solution comprenant au moins un précurseur de la phase active de nickel imprégné sur le support imprégné maturé obtenu à l'issue de l'étape b) est tel que le V2 = VPT - V1.

Le pH de ladite solution comprenant au moins un précurseur de la phase active de nickel imprégné pourra être modifié par l'ajout éventuel d'un acide ou d'une base.

De manière préférée, ledit précurseur de nickel est introduit en solution aqueuse, par exemple sous forme de nitrate, de carbonate, d'acétate, de chlorure, d'oxalate, de complexes formés par un polyacide ou un acide-alcool et ses sels, de complexes formés avec les acétylacétonates, ou de tout autre dérivé inorganique soluble en solution aqueuse, laquelle est mise en contact avec ledit support. De manière préférée, on utilise avantageusement comme précurseur de nickel, le nitrate de nickel, le chlorure de nickel, l'acétate de nickel ou le hydroxycarbonate de nickel. De manière très préférée, le précurseur de nickel est le nitrate de nickel.

La concentration en nickel en solution est ajustée selon le volume poreux du support encore disponible de façon à obtenir pour le catalyseur supporté, une teneur en nickel comprise entre 1 et 50 % poids en élément nickel par rapport au poids total du catalyseur, plus préférentiellement entre 2 et 40 % poids et encore plus préférentiellement entre 3 et 35 % poids et encore plus préférentiellement 5 et 25 % poids.

### Etape d)

L'étape d) de séchage est réalisée avantageusement à une température inférieure à 250°C, de préférence comprise entre 15°C et 180°C, plus préférentiellement entre 30°C et 160°C, encore plus préférentiellement entre 50°C et 150°C, et de manière encore plus préférentielle entre 70°C et 140°C, pendant une durée typiquement comprise entre 0,5 heure à 12 heures, et de façon encore plus préférée pendant une durée de 0,5 heure à 5 heures. Des durées plus longues ne sont pas exclues, mais n'apportent pas nécessairement d'amélioration.

L'étape de séchage peut être effectuée par toute technique connue de l'Homme du métier. Elle est avantageusement effectuée sous une atmosphère inerte ou sous une atmosphère contenant de l'oxygène ou sous un mélange de gaz inerte et d'oxygène. Elle est avantageusement effectuée à pression atmosphérique ou à pression réduite. De manière préférée, cette étape est réalisée à pression atmosphérique et en présence d'air ou d'azote.

A l'issue de l'étape d), la présence totale, partielle, ou l'absence de la solution de butanol dans le catalyseur n'a pas d'incidence sur l'activité et/ou la sélectivité du catalyseur dans le cadre de l'hydrogénation sélective de composés polyinsaturés ou l'hydrogénation de composés aromatiques.

### Etape e) (optionnelle)

L'étape e) de calcination peut être réalisée à une température comprise entre 250°C et 600°C, de préférence entre 350°C et 550°C, pendant une durée typiquement comprise entre 0,5 heure à 24 heures, de façon préférée pendant une durée de 0,5 heure à 12 heures, et de façon encore plus préférée pendant une durée de 0,5 heure à 10 heures, de préférence sous une atmosphère inerte ou sous une atmosphère contenant de l'oxygène. Des durées plus longues ne sont pas exclues, mais n'apportent pas nécessairement d'amélioration.

A l'issue de l'étape e), la présence totale, partielle, ou l'absence de la solution de butanol dans le catalyseur n'a pas d'incidence sur l'activité et/ou la sélectivité du catalyseur dans le cadre de l'hydrogénation sélective de composés polyinsaturés ou l'hydrogénation de composés aromatiques.

### Etape f) (optionnelle)

Préalablement à l'utilisation du catalyseur dans le réacteur catalytique et la mise en oeuvre d'un procédé d'hydrogénation, on effectue avantageusement au moins une étape de traitement réducteur f) en présence d'un gaz réducteur après les étapes d) ou e) de manière à obtenir un catalyseur comprenant du nickel au moins partiellement sous forme métallique.

Ce traitement permet d'activer ledit catalyseur et de former des particules métalliques, en particulier du nickel à l'état zéro valent. Ledit traitement réducteur peut être réalisé *in-situ* ou *ex-situ* c'est-à-dire après ou avant le chargement du catalyseur dans le réacteur d'hydrogénation.

Le gaz réducteur est de préférence l'hydrogène. L'hydrogène peut être utilisé pur ou en mélange (par exemple un mélange hydrogène / azote, ou hydrogène / argon, ou hydrogène / méthane). Dans le cas où l'hydrogène est utilisé en mélange, toutes les proportions sont envisageables.

Ledit traitement réducteur est réalisé à une température comprise entre 120°C et 500°C, de préférence entre 150°C et 450°C. Lorsque le catalyseur ne subit pas de passivation, ou subit un traitement réducteur avant passivation, le traitement réducteur est effectué à une température comprise entre 180°C et 500°C, de préférence entre 200°C et 450°C, et encore plus préférentiellement entre 350°C et 450°C. Lorsque le catalyseur a subi au préalable une passivation, le traitement réducteur est généralement effectué à une température comprise entre 120°C et 350°C, de préférence entre 150°C et 350°C.

La durée du traitement réducteur est généralement comprise entre 2 heures et 40 heures, de préférence entre 3 heures et 30 heures. La montée en température jusqu'à la température de réduction désirée est généralement lente, par exemple fixée entre 0,1°C/min et 10°C/min, de préférence entre 0,3°C/min et 7°C/min.

Le débit d'hydrogène, exprimé en L/heure/gramme de catalyseur est compris entre 0,01 et 100 L/heure/gramme de catalyseur, de préférence entre 0,05 et 10 L/heure/gramme de catalyseur, de façon encore plus préférée entre 0,1 et 5 L/heure/gramme de catalyseur.

### 3. Catalyseur

Le procédé de préparation selon l'invention permet d'obtenir sur un catalyseur comprenant, une phase active à base de nickel et un support d'alumine, ledit catalyseur comprenant entre 1 et 50 % poids de nickel élémentaire par rapport au poids total du catalyseur, le nickel étant réparti à la fois sur une croûte en périphérie du support, et à cœur du support, l'épaisseur de ladite croûte (appelée aussi ep1) étant comprise entre 2% et 15% du diamètre du catalyseur, et la taille des particules de nickel, mesurée sous forme oxyde, dans le catalyseur est comprise entre 7 nm et 25 nm.

De préférence, le nickel est réparti à la fois sur une croûte en périphérie du support, et à cœur du support, l'épaisseur de ladite croûte ep1 étant comprise entre 2% et 15% du diamètre du catalyseur, de préférence entre 2,5% et 12% du diamètre du catalyseur, de façon encore plus préférée entre 3% et 10% du diamètre du catalyseur et de façon encore plus préférée entre 3% et 7,5% du diamètre du catalyseur.

De préférence, le ratio de densité en nickel entre la croûte et le cœur (appelé aussi ici d_{croute}/d_{coeur}) est supérieur strictement à 3, de préférence supérieur à 3,5 et de préférence compris entre 3,8 et 15.

De préférence, ladite croûte comprend plus de 25% en poids d'élément nickel par rapport au poids total d'élément nickel contenu dans le catalyseur, de préférence plus de 40% en poids, plus préférentiellement entre 45% et 90% en poids, et encore plus préférentiellement entre 60% et 90% en poids.

Avantageusement, l'intervalle de transition entre le cœur et la croûte du catalyseur (appelé aussi ici intervalle de transition cœur/croûte, ou ep2-ep1 d'après les notations de la figure 1), lié à la variation de la densité de nickel mesurée sur l'épaisseur du catalyseur depuis le bord du catalyseur jusqu'au centre du catalyseur, est très abrupte. De préférence, l'intervalle de transition cœur/croûte est compris entre 0,05 % et 3 % du diamètre du catalyseur, de préférence entre 0,5 % et 2,5 % du diamètre du catalyseur.

La teneur en nickel dans ledit catalyseur est avantageusement comprise entre 1 et 50 % poids par rapport au poids total du catalyseur, plus préférentiellement entre 2 et 40 % poids et encore plus préférentiellement entre 3 et 35 % poids et encore plus préférentiellement 5 et 25% poids par rapport au poids total du catalyseur. Les valeurs « % poids » se basent sur la forme élémentaire du nickel.

Le catalyseur peut être qualifié comme catalyseur « semi egg-shell » dans lequel la concentration du nickel est plus élevée en périphérie du support que dans le cœur du support, ladite concentration du nickel dans le cœur du support étant non nulle.

La surface spécifique du catalyseur est généralement comprise entre 10 m²/g et 350 m²/g, de préférence entre 25 m²/g et 300 m²/g, de façon plus préférée entre 40 m²/g et 250 m²/g.

Le volume poreux total du catalyseur est généralement compris entre 0,1 ml/g et 1 ml/g, de préférence compris entre 0,2 ml/g et 0,8 ml/g, et de manière particulièrement préférée compris entre 0,3 ml/g et 0,7 ml/g.

La taille des particules de nickel, mesurée sous forme oxyde, dans le catalyseur est comprise entre 7 nm et 25 nm, de préférence entre 8 nm et 23 nm.

De préférence, la phase active du catalyseur ne comprend pas de métal du groupe VIB. Elle ne comprend notamment pas de molybdène ou de tungstène.

Ledit catalyseur (et le support utilisé pour la préparation du catalyseur) est sous forme de grains ayant avantageusement un diamètre compris entre 0,5 mm et 10 mm. Les grains peuvent avoir toutes les formes connues de l'Homme du métier, par exemple la forme de billes (ayant de préférence un diamètre compris entre 1 mm et 8 mm), d'extrudés, de tablettes, de cylindres creux. De préférence, le catalyseur (et le support utilisé pour la préparation du catalyseur) sont sous forme d'extrudés de diamètre compris entre 0,5 mm et 10 mm, de préférence entre 0,8 mm et 3,2 mm et de manière très préférée entre 1,0 mm et 2,5 mm et de longueur comprise entre 0,5 et 20 mm. On entend par « diamètre» des extrudés le diamètre du cercle circonscrit à la section droite de ces extrudés. Le catalyseur peut être avantageusement présenté sous la forme d'extrudés cylindriques, multilobés, trilobés ou quadrilobés. De préférence sa forme est trilobée ou quadrilobée. La forme des lobes peut être ajustée selon toutes les méthodes connues de l'art antérieur.

### 4. Support

Les caractéristiques de l'alumine, mentionnées dans cette section, correspondent aux caractéristiques de l'alumine avant la réalisation de l'étape a) du procédé de préparation selon l'invention.

Le support est une alumine c'est-à-dire que le support comporte au moins 95%, de préférence au moins 98%, et de manière particulièrement préférée au moins 99% poids d'alumine par rapport au poids du support. L'alumine présente généralement une structure cristallographique du type alumine delta, gamma ou thêta, seule ou en mélange.

Le support d'alumine, peut comprendre des impuretés telles que les oxydes de métaux des groupes IIA, IIIB, IVB, IIB, IIIA, IVA selon la classification CAS, de préférence la silice, le dioxyde de titane, le dioxyde de zirconium, l'oxyde de zinc, l'oxyde de magnésium et l'oxyde de calcium, ou encore des métaux alcalins, de préférence le lithium, le sodium ou le potassium, et/ou les alcalino-terreux, de préférence le magnésium, le calcium, le strontium ou le baryum ou encore du soufre.

La surface spécifique de l'alumine est généralement comprise entre 10 m²/g et 400m²/g, de préférence entre 30 m²/g et 350 m²/g, de façon plus préférée entre 50 m²/g et 300m²/g.

Le volume poreux total de l'alumine est généralement compris entre 0,1 ml/g et 1,2 ml/g, de préférence compris entre 0,3 ml/g et 0,9 ml/g, et de manière très préférée compris entre 0,5 ml/g et 0,9 ml/g.

### 5. Procédé d'hydrogénation sélective

Il est également décrit un procédé d'hydrogénation sélective de composés polyinsaturés contenant au moins 2 atomes de carbone par molécule, tels que les dioléfines et/ou les acétyléniques et/ou les alcénylaromatiques, aussi appelés styréniques, contenus dans une charge d'hydrocarbures ayant un point d'ébullition final inférieur ou égal à 300°C, lequel procédé étant réalisé à une température comprise entre 0 et 300°C, à une pression comprise entre 0,1 MPa et 10 MPa, à un ratio molaire hydrogène/(composés polyinsaturés à hydrogéner) compris entre 0,1 et 10 et à une vitesse volumique horaire comprise entre 0,1 h⁻¹ et 200 h⁻¹ lorsque le procédé est réalisé en phase liquide, ou à un ratio molaire hydrogène/(composés polyinsaturés à hydrogéner) compris entre 0,5 et 1000 et à une vitesse volumique horaire entre 100 h⁻¹ et 40000 h⁻¹ lorsque le procédé est réalisé en phase gazeuse, en présence d'un catalyseur obtenu par le procédé de préparation tel que décrit ci-avant dans la description.

Les composés organiques mono-insaturés tels que par exemple l'éthylène et le propylène, sont à la source de la fabrication de polymères, de matières plastiques et d'autres produits chimiques à valeur ajoutée. Ces composés sont obtenus à partir du gaz naturel, du naphta ou du gazole qui ont été traités par des procédés de vapocraquage ou de craquage catalytique. Ces procédés sont opérés à haute température et produisent, en plus des composés mono-insaturés recherchés, des composés organiques polyinsaturés tels que l'acétylène, le propadiène et le méthylacétylène (ou propyne), le 1-2-butadiène et le 1-3-butadiène, le vinylacétylène et l'éthylacétylène, et d'autres composés polyinsaturés dont le point d'ébullition correspond à la coupe C5+ (composés hydrocarbonés ayant au moins 5 atomes de carbone), en particulier des composés dioléfiniques ou styréniques ou indéniques. Ces composés polyinsaturés sont très réactifs et conduisent à des réactions parasites dans les unités de polymérisation. Il est donc nécessaire de les éliminer avant de valoriser ces coupes.

L'hydrogénation sélective est le principal traitement développé pour éliminer spécifiquement les composés polyinsaturés indésirables de ces charges d'hydrocarbures. Elle permet la conversion des composés polyinsaturés vers les alcènes ou aromatiques correspondants en évitant leur saturation totale et donc la formation des alcanes ou naphtènes correspondants. Dans le cas d'essences de vapocraquage utilisées comme charge, l'hydrogénation sélective permet également d'hydrogéner sélectivement les alcénylaromatiques en aromatiques en évitant l'hydrogénation des noyaux aromatiques.

La charge d'hydrocarbures traitée dans le procédé d'hydrogénation sélective a un point d'ébullition final inférieur ou égal à 300°C et contient au moins 2 atomes de carbone par molécule et comprend au moins un composé polyinsaturé. On entend par « composés polyinsaturés » des composés comportant au moins une fonction acétylénique et/ou au moins une fonction diénique et/ou au moins une fonction alcénylaromatique.

Plus particulièrement, la charge est sélectionnée dans le groupe constitué par une coupe C2 de vapocraquage, une coupe C2-C3 de vapocraquage, une coupe C3 de vapocraquage, une coupe C4 de vapocraquage, une coupe C5 de vapocraquage et une essence de vapocraquage encore appelée essence de pyrolyse ou coupe C5+.

La coupe C2 de vapocraquage, avantageusement utilisée pour la mise en œuvre du procédé d'hydrogénation sélective selon l'invention, présente par exemple la composition suivante : entre 40 et 95 % poids d'éthylène, de l'ordre de 0,1 à 5 % poids d'acétylène, le reste étant essentiellement de l'éthane et du méthane. Dans certaines coupes C2 de vapocraquage, entre 0,1 et 1 % poids de composés en C3 peut aussi être présent.

La coupe C3 de vapocraquage, avantageusement utilisée pour la mise en œuvre du procédé d'hydrogénation sélective selon l'invention, présente par exemple la composition moyenne suivante : de l'ordre de 90 % poids de propylène, de l'ordre de 1 à 8 % poids de propadiène et de méthylacétylène, le reste étant essentiellement du propane. Dans certaines coupes C3, entre 0,1 et 2 % poids de composés en C2 et de composés en C4 peut aussi être présent.

Une coupe C2 - C3 peut aussi être avantageusement utilisée pour la mise en œuvre du procédé d'hydrogénation sélective selon l'invention. Elle présente par exemple la composition suivante : de l'ordre de 0,1 à 5 % poids d'acétylène, de l'ordre de 0,1 à 3 % poids de propadiène et de méthylacétylène, de l'ordre de 30 % poids d'éthylène, de l'ordre de 5 % poids de propylène, le reste étant essentiellement du méthane, de l'éthane et du propane. Cette charge peut aussi contenir entre 0,1 et 2 % poids de composés en C4.

La coupe C4 de vapocraquage, avantageusement utilisée pour la mise en œuvre du procédé d'hydrogénation sélective selon l'invention, présente par exemple la composition massique moyenne suivante : 1 % poids de butane, 46,5 % poids de butène, 51 % poids de butadiène, 1,3 % poids de vinylacétylène et 0,2 % poids de butyne. Dans certaines coupes C4, entre 0,1 et 2 % poids de composés en C3 et de composés en C5 peut aussi être présent.

La coupe C5 de vapocraquage, avantageusement utilisée pour la mise en œuvre du procédé d'hydrogénation sélective selon l'invention, présente par exemple la composition suivante : 21 % poids de pentanes, 45 % poids de pentènes, 34 % poids de pentadiènes.

L'essence de vapocraquage ou essence de pyrolyse, avantageusement utilisée pour la mise en œuvre du procédé d'hydrogénation sélective selon l'invention, correspond à une coupe hydrocarbonée dont la température d'ébullition est généralement comprise entre 0 et 300°C, de préférence entre 10°C et 250°C. Les hydrocarbures polyinsaturés à hydrogéner présents dans ladite essence de vapocraquage sont en particulier des composés dioléfiniques (butadiène, isoprène, cyclopentadiène...), des composés styréniques (styrène, alpha-méthylstyrène...) et des composés indéniques (indène...). L'essence de vapocraquage comprend généralement la coupe C5-C12 avec des traces de C3, C4, C13, C14, C15 (par exemple entre 0,1 et 3% poids pour chacune de ces coupes). Par exemple, une charge formée d'essence de pyrolyse a généralement une composition suivante: 5 à 30 % poids de composés saturés (paraffines et naphtènes), 40 à 80 % poids de composés aromatiques, 5 à 20 % poids de mono-oléfines, 5 à 40 % poids de dioléfines, 1 à 20 % poids de composés alcénylaromatiques, l'ensemble des composés formant 100 %. Elle contient également de 0 à 1000 ppm poids de soufre, de préférence de 0 à 500 ppm poids de soufre.

De manière préférée, la charge d'hydrocarbures polyinsaturés traitée conformément au procédé d'hydrogénation sélective selon l'invention est une coupe C2 de vapocraquage, ou une coupe C2-C3 de vapocraquage, ou une essence de vapocraquage.

Le procédé d'hydrogénation sélective selon l'invention vise à éliminer lesdits hydrocarbures polyinsaturés présents dans ladite charge à hydrogéner sans hydrogéner les hydrocarbures monoinsaturés. Par exemple, lorsque ladite charge est une coupe C2, le procédé d'hydrogénation sélective vise à hydrogéner sélectivement l'acétylène. Lorsque ladite charge est une coupe C3, le procédé d'hydrogénation sélective vise à hydrogéner sélectivement le propadiène et le méthylacétylène. Dans le cas d'une coupe C4, on vise à éliminer le butadiène, le vinylacétylène (VAC) et le butyne, dans le cas d'une coupe C5, on vise à éliminer les pentadiènes. Lorsque ladite charge est une essence de vapocraquage, le procédé d'hydrogénation sélective vise à hydrogéner sélectivement lesdits hydrocarbures polyinsaturés présents dans ladite charge à traiter de manière à ce que les composés dioléfiniques soient partiellement hydrogénés en mono-oléfines et que les composés styréniques et indéniques soient partiellement hydrogénés en composés aromatiques correspondants en évitant l'hydrogénation des noyaux aromatiques.

La mise en œuvre technologique du procédé d'hydrogénation sélective est par exemple réalisée par injection, en courant ascendant ou descendant, de la charge d'hydrocarbures polyinsaturés et de l'hydrogène dans au moins un réacteur à lit fixe. Ledit réacteur peut être de type isotherme ou de type adiabatique. Un réacteur adiabatique est préféré. La charge d'hydrocarbures polyinsaturés peut avantageusement être diluée par une ou plusieurs ré-injection(s) de l'effluent, issu dudit réacteur où se produit la réaction d'hydrogénation sélective, en divers points du réacteur, situés entre l'entrée et la sortie du réacteur afin de limiter le gradient de température dans le réacteur. La mise en œuvre technologique du procédé d'hydrogénation sélective selon l'invention peut également être avantageusement réalisée par l'implantation d'au moins dudit catalyseur supporté dans une colonne de distillation réactive ou dans des réacteurs - échangeurs ou dans un réacteur de type slurry. Le flux d'hydrogène peut être introduit en même temps que la charge à hydrogéner et/ou en un ou plusieurs points différents du réacteur.

L'hydrogénation sélective des coupes C2, C2-C3, C3, C4, C5 et C5+ de vapocraquage peut être réalisée en phase gazeuse ou en phase liquide, de préférence en phase liquide pour les coupes C3, C4, C5 et C5+ et en phase gazeuse pour les coupes C2 et C2-C3. Une réaction en phase liquide permet d'abaisser le coût énergétique et d'augmenter la durée de cycle du catalyseur.

D'une manière générale, l'hydrogénation sélective d'une charge d'hydrocarbures contenant des composés polyinsaturés contenant au moins 2 atomes de carbone par molécule et ayant un point d'ébullition final inférieur ou égal à 300°C s'effectue à une température comprise entre 0 et 300°C, à une pression comprise entre 0,1 MPa et 10 MPa, à un ratio molaire hydrogène/(composés polyinsaturés à hydrogéner) compris entre 0,1 et 10 et à une vitesse volumique horaire (définie comme le rapport du débit volumique de charge sur le volume du catalyseur) comprise entre 0,1 h⁻¹ et 200 h⁻¹ pour un procédé réalisé en phase liquide, ou à un ratio molaire hydrogène/(composés polyinsaturés à hydrogéner) compris entre 0,5 et 1000 et à une vitesse volumique horaire comprise entre 100 h⁻¹ et 40000 h⁻¹ pour un procédé réalisé en phase gazeuse.

Dans un mode de réalisation selon l'invention, lorsqu'on effectue un procédé d'hydrogénation sélective dans lequel la charge est une essence de vapocraquage comportant des composés polyinsaturés, le ratio molaire (hydrogène)/(composés polyinsaturés à hydrogéner) est généralement compris entre 0,5 et 10, de préférence entre 0,7 et 5,0 et de manière encore plus préférée entre 1,0 et 2,0, la température est comprise entre 0 et 200°C, de préférence entre 20°C et 200 °C et de manière encore plus préférée entre 30°C et 180°C, la vitesse volumique horaire (V.V.H.) est comprise généralement entre 0,5 h⁻¹ et 100 h⁻¹, de préférence entre 1 h⁻¹ et 50 h⁻¹ et la pression est généralement comprise entre 0,3 et 8,0 MPa, de préférence entre 1,0 MPa et 7,0 MPa et de manière encore plus préférée entre 1,5 MPa et 4,0 MPa.

Plus préférentiellement, on effectue un procédé d'hydrogénation sélective dans lequel la charge est une essence de vapocraquage comportant des composés polyinsaturés, le ratio molaire hydrogène/(composés polyinsaturés à hydrogéner) est compris entre 0,7 et 5,0, la température est comprise entre 20°C et 200 °C, la vitesse volumique horaire (V.V.H.) est comprise généralement entre 1 h⁻¹ et 50 h⁻¹ et la pression est comprise entre 1,0 MPa et 7,0 MPa.

Encore plus préférentiellement, on effectue un procédé d'hydrogénation sélective dans lequel la charge est une essence de vapocraquage comportant des composés polyinsaturés, le ratio molaire hydrogène/(composés polyinsaturés à hydrogéner) est compris entre 1,0 et 2,0, la température est comprise entre 30°C et 180°C, la vitesse volumique horaire (V.V.H.) est comprise généralement entre 1 h⁻¹ et 50 h⁻¹ et la pression est comprise entre 1,5 MPa et 4,0 MPa.

Le débit d'hydrogène est ajusté afin d'en disposer en quantité suffisante pour hydrogéner théoriquement l'ensemble des composés polyinsaturés et de maintenir un excès d'hydrogène en sortie de réacteur.

Dans un autre mode de réalisation selon l'invention, lorsqu'on effectue un procédé d'hydrogénation sélective dans lequel la charge est une coupe C2 de vapocraquage et/ou une coupe C2-C3 de vapocraquage comportant des composés polyinsaturés, le ratio molaire (hydrogène)/(composés polyinsaturés à hydrogéner) est généralement compris entre 0,5 et 1000, de préférence entre 0,7 et 800, la température est comprise entre 0 et 300°C, de préférence entre 15°C et 280 °C, la vitesse volumique horaire (V.V.H.) est comprise généralement entre 100 h⁻¹ et 40000 h⁻¹, de préférence entre 500 h⁻¹ et 30000 h⁻¹ et la pression est généralement comprise entre 0,1 MPa et 6,0 MPa, de préférence entre 0,2 MPa et 5,0 MPa.

### 6. Procédé d'hydrogénation des aromatiques

Il est également décrit un procédé d'hydrogénation d'au moins un composé aromatique ou polyaromatique contenu dans une charge d'hydrocarbures ayant un point d'ébullition final inférieur ou égal à 650°C, généralement compris entre 20°C et 650°C, et de préférence compris entre 20°C et 450°C. Ladite charge d'hydrocarbures contenant au moins un composé aromatique ou polyaromatique peut être choisi parmi les coupes pétrolières ou pétrochimiques suivantes : le reformat du reformage catalytique, le kérosène, le gazole léger, le gazole lourd, les distillats de craquage, tels que l'huile de recyclage de FCC, le gazole d'unité de cokéfaction, les distillats d'hydrocraquage.

La teneur en composés aromatiques ou polyaromatiques contenus dans la charge d'hydrocarbures traitée dans le procédé d'hydrogénation selon l'invention est généralement compris entre 0,1 et 80% en poids, de préférence entre 1 et 50% en poids, et de manière particulièrement préférée entre 2 et 35% en poids, le pourcentage étant basé sur le poids total de la charge d'hydrocarbures. Les composés aromatiques présents dans ladite charge d'hydrocarbures sont par exemple le benzène ou des alkylaromatiques tels que le toluène, l'éthylbenzène, l'o-xylène, le m-xylène, ou le p-xylène, ou encore des aromatiques ayant plusieurs noyaux aromatiques (polyaromatiques) tels que le naphtalène.

La teneur en soufre ou en chlore de la charge est généralement inférieure à 5000 ppm poids de soufre ou de chlore, de préférence inférieure à 100 ppm poids, et de manière particulièrement préférée inférieure à 10 ppm poids.

La mise en œuvre technologique du procédé d'hydrogénation des composés aromatiques ou polyaromatiques est par exemple réalisée par injection, en courant ascendant ou descendant, de la charge d'hydrocarbures et de l'hydrogène dans au moins un réacteur à lit fixe. Ledit réacteur peut être de type isotherme ou de type adiabatique. Un réacteur adiabatique est préféré. La charge d'hydrocarbures peut avantageusement être diluée par une ou plusieurs ré-injection(s) de l'effluent, issu dudit réacteur où se produit la réaction d'hydrogénation des aromatiques, en divers points du réacteur, situés entre l'entrée et la sortie du réacteur afin de limiter le gradient de température dans le réacteur. La mise en oeuvre technologique du procédé d'hydrogénation des aromatiques selon l'invention peut également être avantageusement réalisée par l'implantation d'au moins dudit catalyseur supporté dans une colonne de distillation réactive ou dans des réacteurs - échangeurs ou dans un réacteur de type slurry. Le flux d'hydrogène peut être introduit en même temps que la charge à hydrogéner et/ou en un ou plusieurs points différents du réacteur.

L'hydrogénation des composés aromatiques ou polyaromatiques peut être réalisée en phase gazeuse ou en phase liquide, de préférence en phase liquide. D'une manière générale, l'hydrogénation des composés aromatiques ou polyaromatiques s'effectue à une température comprise entre 30°C et 350°C, de préférence entre 50°C et 325°C, à une pression comprise entre 0,1 MPa et 20 MPa, de préférence entre 0,5 MPa et 10 MPa, à un ratio molaire hydrogène/(composés aromatiques à hydrogéner) entre 0,1 et 10 et à une vitesse volumique horaire comprise entre 0,05 h⁻¹ et 50 h⁻¹, de préférence entre 0,1 h⁻¹ et 10 h⁻¹ d'une charge d'hydrocarbures contenant des composés aromatiques ou polyaromatiques et ayant un point d'ébullition final inférieur ou égal à 650°C, généralement compris entre 20°C et 650°C, et de préférence compris entre 20°C et 450°C.

Le débit d'hydrogène est ajusté afin d'en disposer en quantité suffisante pour hydrogéner théoriquement l'ensemble des composés aromatiques et de maintenir un excès d'hydrogène en sortie de réacteur.

La conversion des composés aromatiques ou polyaromatiques est généralement supérieure à 20% en mole, de préférence supérieure à 40% en mole, de manière plus préférée supérieure à 80% en mole, et de manière particulièrement préférée supérieure à 90 % en mole des composés aromatiques ou polyaromatiques contenus dans la charge hydrocarbonée. La conversion se calcule en divisant la différence entre les moles totales des composés aromatiques ou polyaromatiques dans la charge d'hydrocarbures et dans le produit par les moles totales des composés aromatiques ou polyaromatiques dans la charge d'hydrocarbures.

Selon une variante particulière du procédé selon l'invention, on réalise un procédé d'hydrogénation du benzène d'une charge d'hydrocarbures, tel que le reformat issu d'une unité de reformage catalytique. La teneur en benzène dans ladite charge d'hydrocarbures est généralement comprise entre 0,1 et 40% poids, de préférence entre 0,5 et 35% poids, et de manière particulièrement préférée entre 2 et 30% poids, le pourcentage en poids étant basé sur le poids total de la charge d'hydrocarbures.

La teneur en soufre ou en chlore de la charge est généralement inférieure à 10 ppm poids de soufre ou chlore respectivement, et de préférence inférieure à 2 ppm poids.

L'hydrogénation du benzène contenu dans la charge d'hydrocarbures peut être réalisée en phase gazeuse ou en phase liquide, de préférence en phase liquide. Lorsqu'elle est réalisée en phase liquide, un solvant peut être présent, tel que le cyclohexane, l'heptane, l'octane. D'une manière générale, l'hydrogénation du benzène s'effectue à une température comprise entre 30°C et 250°C, de préférence entre 50°C et 200°C, et de manière plus préférée entre 80°C et 180°C, à une pression comprise entre 0,1 MPa et 10 MPa, de préférence entre 0,5 et 4 MPa, à un ratio molaire hydrogène/(benzène) entre 0,1 et 10 et à une vitesse volumique horaire comprise entre 0,05 h⁻¹ et 50 h⁻¹, de préférence entre 0,5 h⁻¹ et 10 h⁻¹.

La conversion du benzène est généralement supérieure à 50% en mole, de préférence supérieure à 80% en mole, de manière plus préférée supérieure à 90% en mole et de manière particulièrement préférée supérieure à 98 % en mole.

L'invention est illustrée via les exemples ci-après qui ne sont nullement limitatifs.

### Exemples

Pour tous les catalyseurs mentionnés dans les exemples ci-après, le support est une alumine A présentant une surface spécifique de 80 m²/g, un volume poreux total VPT de 0,7 mL/g et un diamètre médian mésoporeux de 12 nm.

### Exemple 1 : Préparation d'une solution aqueuse de précurseur de Ni

La solution aqueuse de précurseurs de Ni (solution S) utilisée pour la préparation des catalyseurs A à G est préparée en dissolvant 43,5 grammes (g) de nitrate de nickel (NiNO₃, fournisseur Strem Chemicals^{®}) dans un volume de 13 mL d'eau distillée. On obtient la solution S dont la concentration en Ni est de 350 g de Ni par litre de solution.

### Exemple 2 : Préparation d'un catalyseur A selon invention [10% poids de Ni - ButOH 25% VPT, en pré-imprégnation]

10 g d'alumine A sont imprégnés avec 2,4 ml de n-butanol ajouté au goutte à goutte. Le support imprégné est ensuite laissé à maturer pendant 30 min à 60°C. Ensuite, 7,1 ml de la solution S préparée à l'exemple 1 est imprégnée goutte-à-goutte sur le support imprégné. Le précurseur de catalyseur ainsi obtenu est ensuite séché en étuve pendant 12 heures à 120°C, puis calciné sous un flux d'air sec de 1 L/h/g de catalyseur à 450°C pendant 2 heures.

On obtient le catalyseur A contenant 10 % en poids de l'élément nickel par rapport au poids total du catalyseur.

Les caractéristiques du catalyseur A ainsi obtenu sont reportées dans le tableau 1 ci-après.

### Exemple 3 : Préparation d'un catalyseur B selon invention [5% poids de Ni - ButOH 25% VPT en pré-imprégnation]

10 g d'alumine A sont imprégnés avec 2,4 ml de n-butanol ajouté au goutte à goutte. Le support imprégné est ensuite laissé à maturer pendant 30 min à 60°C. Ensuite, 3,55 ml la solution S préparée à l'exemple 1 diluée avec de l'eau afin de compléter à 7,1 ml est imprégnée goutte-à-goutte sur le support imprégné. Le précurseur de catalyseur ainsi obtenu est ensuite séché en étuve pendant 12 heures à 120°C, puis calciné sous un flux d'air sec de 1 L/h/g de catalyseur à 450°C pendant 2 heures.

On obtient le catalyseur B contenant 5 % en poids de l'élément nickel par rapport au poids total du catalyseur.

Les caractéristiques du catalyseur B ainsi obtenu sont reportées dans le tableau 1 ci-après.

### Exemple 4 : Préparation d'un catalyseur C selon invention [10% poids de Ni - ButOH 75% VPT en pré-imprégnation]

10 g d'alumine A sont imprégnés avec 7,2 ml de n-butanol ajouté au goutte à goutte. Le support imprégné est ensuite laissé à maturer pendant 30 min à 60°C. Ensuite, 2,4 ml de la solution S préparée à l'exemple 1 est imprégnée goutte-à-goutte sur le support imprégné. Le précurseur de catalyseur ainsi obtenu est ensuite séché en étuve pendant 12 heures à 120°C, puis calciné sous un flux d'air sec de 1 L/h/g de catalyseur à 450°C pendant 2 heures.

On obtient le catalyseur C contenant 10 % en poids de l'élément nickel par rapport au poids total du catalyseur.

Les caractéristiques du catalyseur C ainsi obtenu sont reportées dans le tableau 1 ci-après.

### Exemple 5 : Préparation d'un catalyseur D non-conforme à l'invention [imprégnation classique 10%Ni]

La solution S préparée à l'exemple 1 est imprégnée à sec, en l'ajoutant goutte-à-goutte, sur 10 g d'alumine. Le précurseur de catalyseur ainsi obtenu est ensuite séché en étuve pendant 12 heures à 120°C, puis calciné sous un flux d'air sec de 1 L/h/g de catalyseur à 450°C pendant 2 heures.

On obtient le catalyseur D contenant 10 % en poids de l'élément nickel par rapport au poids total du catalyseur.

Les caractéristiques du catalyseur D ainsi obtenu sont reportées dans le tableau 1 ci-après.

### Exemple 6 : Préparation d'un catalyseur E non-conforme [10% poids de Ni - ButOH 25% VPT en post-imprégnation]

7,1 ml de la solution S préparée à l'exemple 1 est imprégnée à sec, en l'ajoutant goutte-à-goutte, sur 10 g de l'alumine. Les 10 g du précurseur de catalyseur préparé sont imprégnés avec 2,4 ml de n-butanol ajouté au goutte à goutte. Le solide est ensuite laissé à maturé pendant 30 min à 60°C.

Le solide ainsi obtenu est ensuite séché en étuve pendant 12 heures à 120°C, puis calciné sous un flux d'air sec de 1 L/h/g de catalyseur à 450°C pendant 2 heures.

On obtient le catalyseur E contenant 10 % en poids de l'élément nickel par rapport au poids total du catalyseur.

Les caractéristiques du catalyseur E ainsi obtenu sont reportées dans le tableau 1 ci-après.

### Exemple 7 : Préparation d'un catalyseur F non-conforme [10% poids de Ni - Toluène 25% VPT en pré -imprégnation]

10 g d'alumine A sont imprégnés avec 2,4 ml de toluène ajouté au goutte à goutte. Le support imprégné est ensuite laissé à maturer pendant 30 min à 60°C. Ensuite, 7,1 ml de la solution S préparée à l'exemple 1 est imprégnée goutte-à-goutte sur le support imprégné. Le précurseur de catalyseur ainsi obtenu est ensuite séché en étuve pendant 12 heures à 120°C, puis calciné sous un flux d'air sec de 1 L/h/g de catalyseur à 450°C pendant 2 heures.

On obtient le catalyseur F contenant 10 % en poids de l'élément nickel par rapport au poids total du catalyseur.

Les caractéristiques du catalyseur F ainsi obtenu sont reportées dans le tableau 1 ci-après.

### Exemple 8 : Préparation d'un catalyseur G non conforme [10% poids de Ni - n-propanol 25% VPT en pré-imprégnation]

10 g d'alumine A sont imprégnés avec 2,4 ml de n-propanol ajouté au goutte à goutte. Le support imprégné est ensuite laissé à maturer pendant 30 min à 60°C. Ensuite, 7,1 ml de la solution S préparée à l'exemple 1 est imprégnée goutte-à-goutte sur le support imprégné. Le précurseur de catalyseur ainsi obtenu est ensuite séché en étuve pendant 12 heures à 120°C, puis calciné sous un flux d'air sec de 1 L/h/g de catalyseur à 450°C pendant 2 heures.

On obtient le catalyseur G contenant 10 % en poids de l'élément nickel par rapport au poids total du catalyseur.

Les caractéristiques du catalyseur G ainsi obtenu sont reportées dans le tableau 1 ci-après.

**Tableau 1 : Caractéristiques des catalyseurs A à G**

| Catalyseur | Solvant (% en volume par rapport au VPT du support) | Ni (% pds) | Taille des particules (nm) | Epaisseur de croute / diamètre du grain (%) | d_{croute}/ d_{coeur} | Teneur en Ni dans la croûte / Ni total (%) |
|---|---|---|---|---|---|---|
| A (conforme) | 25% BuTOH en pré-imprégnation | 10 | 14,5 | 6,8 | 6 | 68 |
| B (conforme) | 25% BuTOH en pré-imprégnation | 5 | 7 | 3,8 | 12 | 71 |
| C (conforme) | 75% BuTOH en pré-imprégnation | 10 | 14 | 4,7 | 13 | 75 |
| D (non conforme) | - | 10 | 12 | <1% | 1,5 | 7 |
| E (non conforme) | 25% BuTOH en post-imprégnation | 10 | 12 | Répartition homogène | - | - |
| F (non conforme) | 25% toluène en pré-imprégnation | 10 | 11,5 | Répartition homogène | - | - |
| G (non conforme) | 25% n-propanol en pré-imprégnation | 10 | 11,5 | Répartition homogène | - | - |

### Exemple 9 : Tests catalytiques : performances en hydrogénation sélective d'un mélange contenant du styrène et de l'isoprène (A_{HYD1})

Les catalyseurs A à G décrits dans les exemples ci-dessus sont testés vis-à-vis de la réaction d'hydrogénation sélective d'un mélange contenant du styrène et de l'isoprène.

La composition de la charge à hydrogéner sélectivement est la suivante : 8 % pds styrène (fournisseur Sigma Aldrich^{®}, pureté 99%), 8 % pds isoprène (fournisseur Sigma Aldrich^{®}, pureté 99%), 84 % pds n-heptane (solvant) (fournisseur VWR^{®}, pureté > 99% chromanorm HPLC). Cette charge contient également des composés soufrés en très faible teneur : 10 ppm pds de soufre introduits sous forme de pentanethiol (fournisseur Fluka^{®}, pureté > 97%) et 100 ppm pds de soufre introduits sous forme de thiophène (fournisseur Merck^{®}, pureté 99%). Cette composition correspond à la composition initiale du mélange réactionnel. Ce mélange de molécules modèles est représentatif d'une essence de pyrolyse.

La réaction d'hydrogénation sélective est opérée dans un autoclave de 500 mL en acier inoxydable, muni d'une agitation mécanique à entraînement magnétique et pouvant fonctionner sous une pression maximale de 100 bar (10 MPa) et des températures comprises entre 5°C et 200°C.

Préalablement à son introduction dans l'autoclave, une quantité de 3 mL de catalyseur est réduite *ex situ* sous un flux d'hydrogène de 1 L/h/g de catalyseur, à 400 °C pendant 16 heures (rampe de montée en température de 1°C/min), puis elle est transvasée dans l'autoclave, à l'abri de l'air. Après ajout de 214 mL de n-heptane (fournisseur VWR^{®}, pureté > 99% chromanorm HPLC), l'autoclave est fermé, purgé, puis pressurisé sous 35 bar (3,5 MPa) d'hydrogène, et porté à la température du test égale à 30°C. Au temps t=0, environ 30 g d'un mélange contenant du styrène, de l'isoprène, du n-heptane, du pentanethiol et du thiophène sont introduits dans l'autoclave. Le mélange réactionnel a alors la composition décrite ci-dessus et l'agitation est mise en route à 1600 tr/min. La pression est maintenue constante à 35 bar (3,5 MPa) dans l'autoclave à l'aide d'une bouteille réservoir située en amont du réacteur.

L'avancement de la réaction est suivi par prélèvement d'échantillons du milieu réactionnel à intervalles de temps réguliers : le styrène est hydrogéné en éthylbenzène, sans hydrogénation du cycle aromatique, et l'isoprène est hydrogéné en méthyl-butènes. Si la réaction est prolongée plus longtemps que nécessaire, les méthyl-butènes sont à leur tour hydrogénés en isopentane. La consommation d'hydrogène est également suivie au cours du temps par la diminution de pression dans une bouteille réservoir située en amont du réacteur. L'activité catalytique est exprimée en moles de H₂ consommées par minute et par gramme de Ni.

Les activités catalytiques mesurées (A_{HYD1}) pour les catalyseurs A à G sont reportées dans le tableau 2 ci-après. Elles sont rapportées à l'activité catalytique mesurée pour le catalyseur D.

### Exemple 10 : Tests catalytiques : performances en hydrogénation du toluène (A_{HYD2})

Les catalyseurs A à G décrits dans les exemples ci-dessus sont également testés vis-à-vis de la réaction d'hydrogénation du toluène.

La réaction d'hydrogénation sélective est opérée dans le même autoclave que celui décrit à l'exemple 9.

Préalablement à son introduction dans l'autoclave, une quantité de 2 mL de catalyseur est réduite *ex situ* sous un flux d'hydrogène de 1 L/h/g de catalyseur, à 400°C pendant 16 heures (rampe de montée en température de 1°C/min), puis elle est transvasée dans l'autoclave, à l'abri de l'air. Après ajout de 216 mL de n-heptane (fournisseur VWR^{®}, pureté > 99% chromanorm HPLC), l'autoclave est fermé, purgé, puis pressurisé sous 35 bar (3,5 MPa) d'hydrogène, et porté à la température du test égale à 80°C. Au temps t=0, environ 26 g de toluène (fournisseur SDS^{®}, pureté > 99,8%) sont introduits dans l'autoclave (la composition initiale du mélange réactionnel est alors toluène 6 % pds / n-heptane 94 % pds) et l'agitation est mise en route à 1600 tr/min. La pression est maintenue constante à 35 bar (3,5 MPa) dans l'autoclave à l'aide d'une bouteille réservoir située en amont du réacteur.

L'avancement de la réaction est suivi par prélèvement d'échantillons du milieu réactionnel à intervalles de temps réguliers : le toluène est totalement hydrogéné en méthylcyclohexane. La consommation d'hydrogène est également suivie au cours du temps par la diminution de pression dans une bouteille réservoir située en amont du réacteur. L'activité catalytique est exprimée en moles de H₂ consommées par minute et par gramme de Ni.

Les activités catalytiques (A_{HYD2}) mesurées pour les catalyseurs A à G sont reportées dans le tableau 2 ci-après. Elles sont rapportées à l'activité catalytique mesurée pour le catalyseur D.

**Tableau 2 : Comparaison des performances des catalyseurs A à G en hydrogénation sélective d'un mélange contenant du styrène et de l'isoprène (A_{HYD1}) et en hydrogénation du toluène (A_{HYD2})**

| Catalyseur | Teneur en Ni° (%) | AHYD1 (%) | AHYD2 (%) |
|---|---|---|---|
| A (conforme) | 10 | 180 | 210 |
| B (conforme) | 5 | 130 | 150 |
| C (conforme) | 10 | 190 | 230 |
| D (non conforme) | 10 | 100 | 100 |
| E (non conforme) | 10 | 50 | 50 |
| F (non conforme) | 10 | 70 | 80 |
| G (non conforme) | 10 | 95 | 98 |

Ceci montre bien les performances améliorées des catalyseurs A, B et C selon l'invention, par rapport aux catalyseurs D, E, F et G non conformes. Ceci s'explique par la répartition du nickel en croûte sur les catalyseurs A, B, et C qui leur confèrent une activité nettement améliorée notamment dans les réactions rapides d'hydrogénation.

Le catalyseur D est en retrait d'activité du fait de l'imprégnation classique mis en œuvre sans pré-imprégnation de butanol. Le catalyseur E a subit une post-imprégnation au butanol ce qu'il ne permet pas une répartition en croûte du nickel. Le catalyseur F est préparé avec une étape de pré-imprégnation du toluène. Ainsi, bien que le toluène soit peu miscible avec l'eau, comme dans le cas du butanol, l'absence de groupements -OH dans la molécule ne lui permet pas une interaction forte avec les -OH du support d'alumine, qui peut expliquer la migration du toluène par l'eau contenue dans la solution de nitrate de nickel lors de l'étape d'imprégnation du nickel. Dans le cas du propanol, les groupements -OH semblent lui permette bien à la fois d'aller à cœur du support et d'être en interaction avec le support. En revanche, l'eau et le n-propanol étant très miscible contrairement au couple butanol/eau, la diffusion à cœur de la solution aqueuse de nitrate de Ni semble avoir lieu compte tenu à la fois des caractéristiques physico-chimiques du catalyseur final obtenu et des résultats de tests catalytiques. Ainsi, pour les catalyseurs E, F et G, le nickel est réparti de façon homogène dans tout le grain de catalyseur. Les catalyseurs E et F ont dès lors une activité bien en retrait du catalyseur A en A_{HYD1} et en A_{HYD2}. Le catalyseur F est encore plus en retrait du fait de la présence de toluène qui perturbe l'imprégnation de la solution de nitrate de nickel.

## Revendications

1. Procédé de préparation d'un catalyseur comprenant une phase active à base de nickel et un support d'alumine, ledit catalyseur comprenant entre 1 et 50% en poids de nickel élémentaire par rapport au poids total du catalyseur, le nickel étant réparti à la fois sur une croûte en périphérie du support, et à cœur du support, l'épaisseur de ladite croûte mesuré par microsonde de Castaing étant comprise entre 2% et 15% du diamètre du catalyseur, la taille des particules de nickel dans le catalyseur, mesurée sous forme oxyde et déterminé par diffraction des rayons X, étant comprise entre 7 nm et 25 nm, lequel procédé comprend les étapes suivantes :
a) on imprègne ledit support avec un volume V1 d'une solution de butanol compris entre 0,2 et 0,8 fois le volume poreux total VPT dudit support pour obtenir un support imprégné, ledit volume poreux total étant mesuré par porosimétrie au mercure selon la norme ASTM D4284-92 avec un angle de mouillage de 140° ;
b) on laisse maturer le support imprégné obtenu à l'issue de l'étape a) pendant 0,5 heure à 40 heures ;
c) on imprègne le support imprégné maturé obtenu à l'issue de l'étape b) avec une solution comprenant au moins un précurseur de la phase active de nickel pour obtenir un précurseur de catalyseur ;
d) on sèche le précurseur de catalyseur obtenu à l'issue de l'étape c) à une température inférieure à 250°C.

2. Procédé selon la revendication 1, dans lequel à l'étape c) le volume V2 de la solution comprenant au moins un précurseur de la phase active de nickel imprégné sur le support imprégné maturé obtenu à l'issue de l'étape b) est tel que le V2 = VPT - V1.

3. Procédé selon l'une des revendications 1 ou 2, dans lequel l'étape b) est réalisée à une température inférieure ou égale à 60°C.

4. Procédé selon l'une quelconque des revendications 1 à 3, dans lequel à l'étape a) on utilise une solution de n-butanol.

5. Procédé selon l'une quelconque des revendications 1 à 4, **caractérisé en ce que** l'étape d) est réalisée pendant un temps compris entre 0,5 heures et 12 heures.

6. Procédé selon l'une quelconque des revendications 1 à 5, **caractérisé en ce qu'**il comprend en outre une étape e) dans laquelle on calcine le catalyseur obtenu à l'issue de l'étape d) à une température comprise entre 250°C et 600°C.

7. Procédé selon la revendication 6, dans lequel l'étape e) est réalisée pendant 0,5 heure à 24 heures.

8. Procédé selon l'une quelconque des revendications 1 à 7, dans lequel à l'étape a) ledit volume V1 de ladite solution de butanol est compris entre 0,25 et 0,75 fois le volume poreux total VPT dudit support.

9. Procédé selon l'une quelconque des revendications 1 à 8, dans lequel le précurseur de la phase active de nickel est le nitrate de nickel, le chlorure de nickel, l'acétate de nickel ou le hydroxycarbonate de nickel.

10. Procédé selon l'une quelconque des revendications 1 à 9, dans lequel le catalyseur comprend une surface spécifique comprise entre 10 m²/g et 350 m²/g mesurée par physisorption à l'azote selon la norme ASTM D3663-03.

## Patentansprüche

1. Verfahren zur Herstellung eines Katalysators, der eine aktive Phase auf Nickelbasis und einen Aluminiumoxid-Träger umfasst, wobei der Katalysator zwischen 1 und 50 Gew.-% elementares Nickel, bezogen auf das Gesamtgewicht des Katalysators, umfasst, wobei das Nickel sowohl auf einer Schale am Umfang des Trägers als auch im Kern des Trägers verteilt ist, die mit einer Castaing-Mikrosonde gemessene Dicke der Schale zwischen 2 % und 15 % des Durchmessers des Katalysators beträgt, die Größe der Nickelteilchen im Katalysator, die in der Oxidform gemessen und mittels Röntgenbeugung bestimmt wird, zwischen 7 nm und 25 nm beträgt, wobei das Verfahren die folgenden Schritte umfasst:
a) das Tränken des Trägers mit einem Volumen V1 einer Butanollösung mit dem 0,2- bis zum 0,8-Fachen des Gesamtporenvolumens GPV des Trägers, wodurch ein getränkter Träger erhalten wird, wobei das Gesamtporenvolumen mittels Quecksilberporosimetrie gemäß der Norm ASTM D4284-92 mit einem Benetzungswinkel von 140° gemessen wird;
b) das Reifenlassen des aus Schritt a) erhaltenen getränkten Trägers für 0,5 Stunden bis 40 Stunden;
c) das Tränken des nach Schritt b) erhaltenen gereiften getränkten Trägers mit einer Lösung, die mindestens eine Vorstufe der aktiven Nickelphase umfasst, wodurch eine Katalysatorvorstufe erhalten wird;
d) das Trocknen der nach Schritt c) erhaltenen Katalysatorvorstufe bei einer Temperatur unter 250 °C.

2. Verfahren nach Anspruch 1, wobei in Schritt c) das Volumen V2 der mindestens eine Vorstufe der aktiven Nickelphase umfassenden Lösung, mit welcher der nach Schritt b) erhaltene gereifte getränkte Träger getränkt wird, so ist, dass V2 = GPV - V1.

3. Verfahren nach einem der Ansprüche 1 oder 2, wobei Schritt b) bei einer Temperatur von weniger als oder gleich 60 °C durchgeführt wird.

4. Verfahren nach einem der Ansprüche 1 bis 3, wobei in Schritt a) eine n-Butanol-Lösung verwendet wird.

5. Verfahren nach einem der Ansprüche 1 bis 4, **dadurch gekennzeichnet, dass** Schritt d) für einen Zeitraum zwischen 0,5 Stunden und 12 Stunden durchgeführt wird.

6. Verfahren nach einem der Ansprüche 1 bis 5, **dadurch gekennzeichnet, dass** es außerdem einen Schritt e) umfasst, bei dem der nach Schritt d) erhaltene Katalysator bei einer Temperatur zwischen 250 °C und 600 °C calciniert wird.

7. Verfahren nach Anspruch 6, wobei Schritt e) 0,5 Stunden bis 24 Stunden lang durchgeführt wird.

8. Verfahren nach einem der Ansprüche 1 bis 7, wobei in Schritt a) das Volumen V1 der Butanollösung zwischen dem 0,25- und dem 0,75-Fachen des Gesamtporenvolumens GPV des Trägers beträgt.

9. Verfahren nach einem der Ansprüche 1 bis 8, wobei es sich bei der Vorstufe der aktiven Nickelphase um Nickelnitrat, Nickelchlorid, Nickelacetat oder Nickelhydroxycarbonat handelt.

10. Verfahren nach einem der Ansprüche 1 bis 9, wobei der Katalysator eine spezifische Oberfläche zwischen 10 m²/g und 350 m²/g umfasst, gemessen mittels Stickstoff-Physisorption gemäß der Norm ASTM D3663-03.

## Claims

1. Process for the preparation of a catalyst comprising a nickel-based active phase and an alumina support, said catalyst comprising between 1% and 50% by weight of elemental nickel, with respect to the total weight of the catalyst, the nickel being distributed both on a crust at the periphery of the support and at the core of the support, the thickness of said crust, measured by Castaing microprobe, being between 2% and 15% of the diameter of the catalyst, the size of the nickel particles in the catalyst, measured in oxide form and determined by X-ray diffraction, being between 7 nm and 25 nm, which process comprises the following stages:
a) said support is impregnated with a volume V1 of a solution of butanol of between 0.2 and 0.8 times the total pore volume TPV of said support, in order to obtain an impregnated support, said total pore volume being measured by mercury porosimetry according to Standard ASTM D4284-92 with a wetting angle of 140°;
b) the impregnated support obtained on conclusion of stage a) is left to mature for 0.5 hour to 40 hours;
c) the matured impregnated support obtained on conclusion of stage b) is impregnated with a solution comprising at least one precursor of the nickel active phase, in order to obtain a catalyst precursor;
d) the catalyst precursor obtained on conclusion of stage c) is dried at a temperature of less than 250°C.

2. Process according to Claim 1, in which, in stage c), the volume V2 of the solution comprising at least one precursor of the nickel active phase impregnated on the matured impregnated support obtained on conclusion of stage b) is such that V2 = TPV - V1.

3. Process according to either of Claims 1 and 2, in which stage b) is carried out at a temperature of less than or equal to 60°C.

4. Process according to any one of Claims 1 to 3, in which, in stage a), a solution of n-butanol is used.

5. Process according to any one of Claims 1 to 4, **characterized in that** stage d) is carried out for a time of between 0.5 hour and 12 hours.

6. Process according to any one of Claims 1 to 5, **characterized in that** it additionally comprises a stage e) in which the catalyst obtained on conclusion of stage d) is calcined at a temperature of between 250°C and 600°C.

7. Process according to Claim 6, in which stage e) is carried out for 0.5 hour to 24 hours.

8. Process according to any one of Claims 1 to 7, in which, in stage a), said volume V1 of said solution of butanol is between 0.25 and 0.75 times the total pore volume TPV of said support.

9. Process according to any one of Claims 1 to 8, in which the precursor of the nickel active phase is nickel nitrate, nickel chloride, nickel acetate or nickel hydroxycarbonate.

10. Process according to any one of Claims 1 to 9, in which the catalyst comprises a specific surface of between 10 m²/g and 350 m²/g, measured by nitrogen physisorption according to Standard ASTM D3663-03.
